# EUROPEAN PATENT SPECIFICATION

(11) **EP 2 788 308 B1**
(45) Date of publication and mention of the grant of the patent: **14.03.2018**
(21) Application number: 12797883.1
(22) Date of filing: 04.12.2012
(51) Int. Cl.: C07C 17/25, C07C 17/383, C07C 21/06, C08F 14/06

(54) **PROCESS FOR THE MANUFACTURE OF VINYL CHLORIDE MONOMER (VCM) AND OF POLYVINYL CHLORIDE (PVC)**
VERFAHREN ZUR HERSTELLUNG VON VINYLCHLORIDMONOMER (VCM) UND POLYVINYLCHLORID (PVC)
PROCÉDÉ POUR LA FABRICATION DE MONOMÈRE DE CHLORURE DE VINYLE ET DE CHLORURE DE POLYVINYLE

(30) Priority: 06.12.2011 EP 11192035
(43) Date of publication of application: 15.10.2014
(73) Proprietor: Solvay SA, 1120 Brussels (BE)
(72) Inventor: SALTO, Andrea, Bahia Blanca 8000 (AR); MARTIN CARNICERO, Maria, 1060 Brussels (BE); DEGRAEVE, Paul Julius, 7822 Isières (BE); LEMPEREUR, Michel, 1435 Corbais (BE)
(74) Representative: Vande Gucht, Anne
(86) International application number: PCT/EP2012/074344
(87) International publication number: WO 2013/083555

(56) References cited:
- WO-A1-2005/003187
- CA-A1- 1 127 669
- DE-A1- 3 147 310

## Description

The present invention relates to a process for the manufacture of vinyl chloride monomer (VCM).

For producing VCM, two methods generally are employed: the hydrochlorination of acetylene and the dehydrochlorination of ethylene dichloride (1,2-dichloroethane) or EDC. The latter generally happens by thermal cracking and the EDC used therefore is generally obtained by direct chlorination and/or oxychlorination of ethylene.

As namely explained in "Chemical Process Design: Computer-Aided Case Studies", Alexandre C. Dimian and Costin Sorin Bildea, Copyright © 2008 WILEY-VCH Verlag GmbH & Co. KGaA, Weinheim, ISBN: 978-3-527-31403-4, Chapter 7 entitled: "Vinyl Chloride Monomer Process", to date, most of the VCM technologies are based on "balanced" processes.

By this is meant that all intermediates and by-products are recycled in a way that ensures a tight closure of the material balance to only VCM as the final product, starting from ethylene, chlorine and oxygen. The main chemical steps involved are:
1. Direct chlorination of ethylene to 1,2 - ethylene dichloride (EDC):

   C2H4+Cl2→C2H4Cl2+218kJ/mol
2. Thermal cracking (pyrolysis) of EDC to VCM:

   C2H4Cl2→C2H3Cl+HCl-71kJ/mol
3. Recovery of HCl and oxychlorination of ethylene to EDC:

   C2H4+2HCl+0.5O2→C2H4Cl2+H2O+238kJ/mol

Hence, an ideal balanced process can be described by the overall equation:
C2H4+0.5Cl2+0.25O2→C2H3Cl+0.5H2O+192.5kJ/mol

As set forth above, the reaction product of the pyrolysis reaction is a gaseous mixture of VCM and HCl and since this reaction is in fact not entirely completed, unconverted EDC is also present in said mixture. This gaseous mixture, which generally is at a high temperature (about 500°C), is rapidly cooled by quenching and then condensed and the gas+liquid mixture so obtained is then subjected to separation, generally by distillation and generally using at least two steps/columns:
Column 1 (or HCl column): feed: (VCM + HCl + EDC) from cracker/top: HCl (+C2H2)/bottom: (VCM + EDC)
Column 2 (or VCM column): feed: (VCM + EDC) from column 1/top: crude VCM/bottom: unconverted EDC.

This same document sets forth, namely in sub-chapter 7.7, several ways of saving energy in a "balanced" process as described above. One of these ways consists in using the enthalpy of the cracker outlet stream after it has been quenched (in order to prevent decomposition of the VCM produced and to remove coke and other impurities) for ensuring the reboiler duty of column 2. This is possible in the process detailed in that document because of the respective temperatures at the outlet stream (139°C) and at reboiler (129°C). However, in many industrial processes, column 2 operates at higher temperature (and pressure) so that this solution cannot be applied.

Patent application CA 1127669 also discloses using the enthalpy of the cracker outlet stream for ensuring the reboiler duty of column 2, but before said stream has been quenched in order to have a sufficient heat (temperature) available for ensuring said duty, considering the fact that said reboiler operates at a temperature of at least 200°C.

The present invention aims at providing a new route for energy saving in a VCM manufacturing process, which also focuses on this VCM column energy consumption, but allows using a stream of lower thermal content.

To this effect, the invention relates to a process for the manufacture of vinyl chloride monomer (VCM), comprising the steps of:
1. subjecting 1,2-dichloroethane (EDC) to pyrolysis in order to generate a gas mixture comprising VCM, HCl and EDC
2. quenching and eventually further cooling and/or condensing said gas mixture to a liquid+gas mixture
3. subjecting said liquid+gas mixture to a first separation step to remove substantially all the HCl there from so as to leave a stream consisting substantially of VCM and EDC
4. subjecting said VCM+EDC stream to a second separation step so as to get a stream of substantially pure VCM and a stream of unconverted EDC, according to which a heat exchanger is used to heat up the VCM+EDC stream prior to being fed to a distillation column in step 4, said heat exchanger being powered by a stream of hot fluid available in any one of steps 2 to 4 of the process but after the quenching of step 2.

In the above, the term « substantially » means in fact that there only remains a limited amount of impurities (typically: a few w% or less) in said streams. As to the terms "a stream of hot fluid available in any one of steps 1 to 4 of the process", they tend to designate any stream of fluid (gas and/or gas+liquid mixture) entering, being inside or leaving any of said steps.

In a first embodiment of the invention, the heat exchanger is powered by at least part of the stream of unconverted EDC obtained in step 4.

In a second embodiment of the invention, the heat exchanger is powered by a stream of hot mixture comprising VCM, HCl and EDC available in step 2 after the quenching.

In step 1 of the process according to the invention, the conditions under which the pyrolysis may be carried out are known to persons skilled in the art. This pyrolysis is advantageously obtained by a reaction in the gaseous phase in a tubular oven. The usual pyrolysis temperatures are between 400 and 600°C with a preference for the range between 480°C and 540°C. The residence time is advantageously between 1 and 60 s with a preference for the range from 5 to 25 s. The rate of conversion of the EDC is advantageously limited to 45 to 75 % in order to limit the formation of by-products and the fouling of the tubes of the oven. Typically, the gas mixture coming from the pyrolysis is at a pressure from 10 to 25 barg.

In step 2 of the process according to the invention, this gas mixture is first cooled down in a quench device (tower generally) and thereafter, generally partially condensed using at least one condenser but preferably, at least 2 or even more preferably: a train of successive condensers. As used herein, "quench device" is a device for removing some components of the gases (namely coke particles that are generally generated during pyrolysis) there from by putting a sufficient quantity of liquid quench medium (generally a liquid mixture of VCM+HCl+EDC recycled from downstream condensation step) in contact with them. After quenching, the temperature of the gases is generally below 200°C, preferably below 180°C and even more preferably, below 150°C. Such a low thermal content would not allow ensuring the thermal duty of the VCM column but it is sufficient to heat up the entry (feed) of said column according to the present invention.

At the end of step 2, when said step also comprises partial condensing, the temperature of the gases is generally comprised between 25 and 50°C and the pressure is adapted between the pressure of step 1 and the operating pressure of the first separation step 3.

In a preferred embodiment of the invention, at least 2 condensers are used having each an inlet and an outlet stream and the heat exchanger is powered by at least part of the inlet stream of the last condenser.

Preferably, the first separation step 3 of the process according to the invention involves a distillation column that separates HCl on top from VCM and EDC at the bottom. This column is preferably operated under a pressure of from 9 to 14 barg. The HCl separated on top can be used in an oxychlorination unit (for instance for making EDC from ethylene) or for any other purpose. A refrigeration unit is preferably used on top of this column to liquefy the HCl required for the reflux of the column. Sieve trays or valves trays can be used in this column.

Preferably, the second separation step 4 of the process according to the invention involves a distillation column that separates VCM on top while unconverted EDC is purged at the bottom. This column is preferably operated under a pressure of from 4 to 8 barg depending on the temperature of the cooling fluid (usually cooling water) available for the condensation on top of the column. VCM required for the reflux of the column and produced VCM are condensed. Sieve trays or valves trays can be used in this column.

According to the invention, the heat exchanger may be of any type. It preferably is a multi-tubular heat exchanger, a spiral heat exchanger or a Compabloc® heat exchanger. Multi-tubular heat exchangers are more particularly preferred.

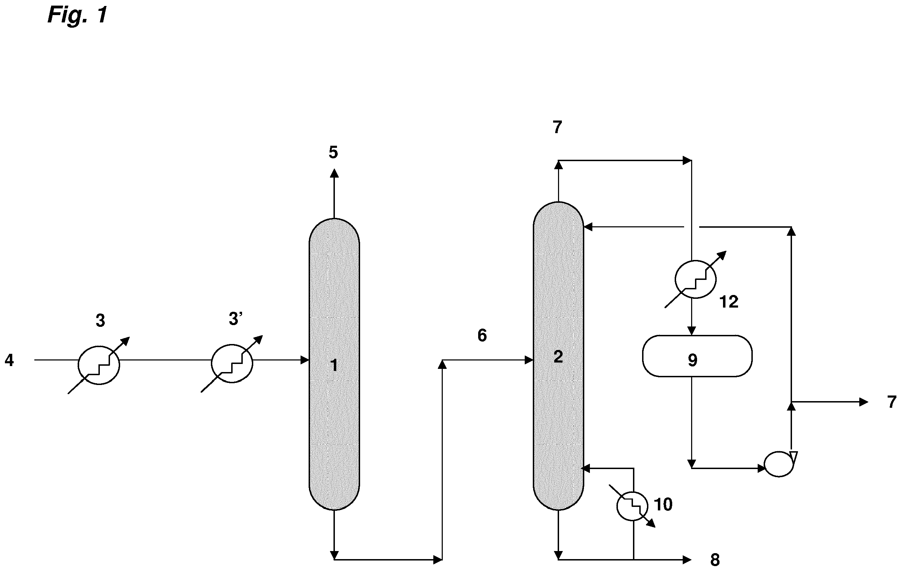

The present invention is illustrated in a non limitative way by figures 1 to 3 attached, which show some preferred embodiments thereof. In these figures, identical reference numbers designate identical or similar items.

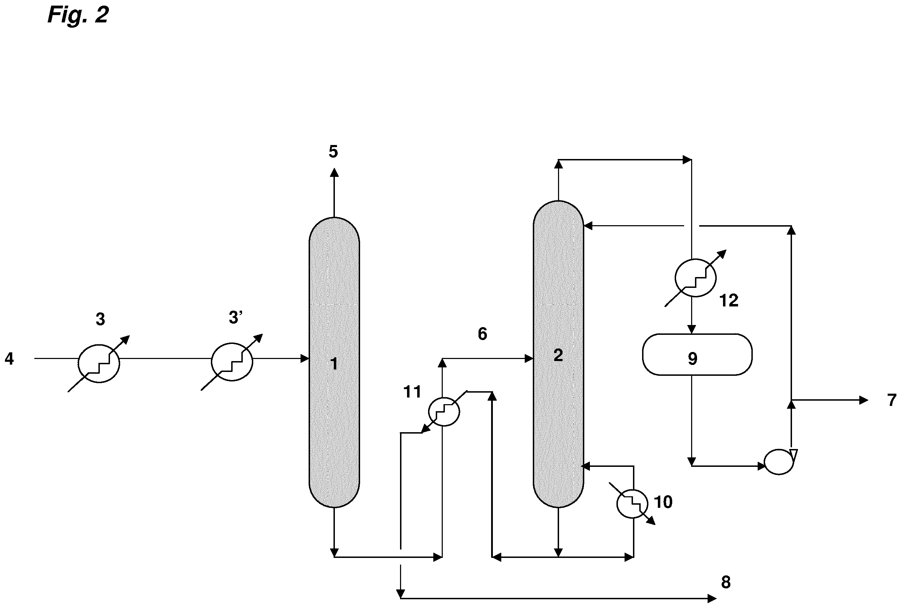

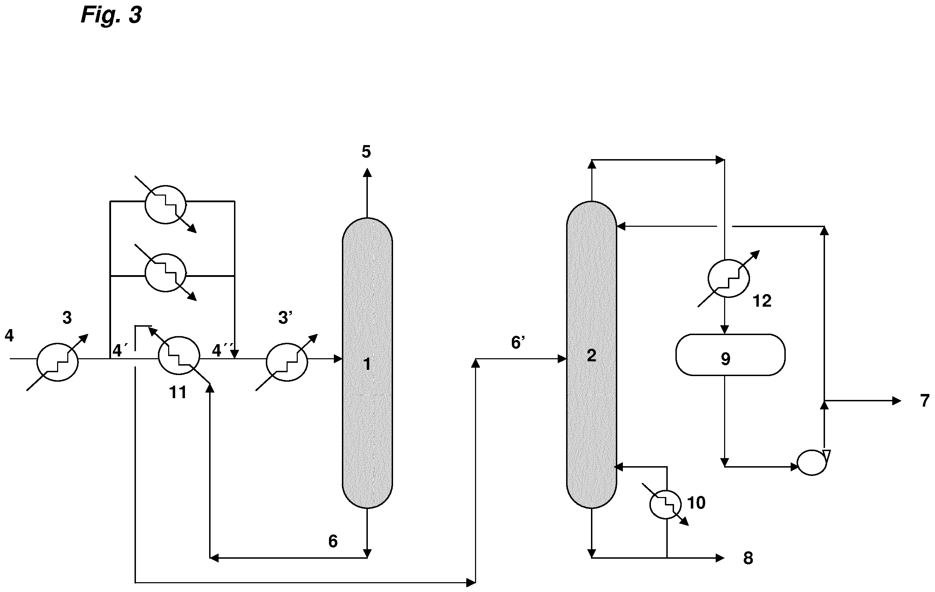

Figure 1 shows a typical arrangement of HCl and VCM columns according to prior art, and figures 2 and 3 show two different embodiments of arrangements according to the invention.

As can be seen from figure 1, a gaseous mixture (4) of (HCl + VCM + EDC) coming from an EDC pyrolysis section and its downstream quench unit (not shown) is first condensed in 2 condensers (3 and 3'), then separated in 2 steps:
- HCl (5) is separated on top of the HCl column (1), and a mixture of (VCM + EDC) (6) is directed to the VCM column (2);
- VCM (7) is separated on top of the VCM column (2);
- unconverted EDC (8) is separated at the bottom of the VCM column and recycled to an EDC purification section (not shown).

In this typical arrangement, the (VCM + EDC) mixture (6) is directly sent to the VCM column (2), which has a VCM condenser (12) and a reflux drum (9) on the VCM stream, and a reboiler (10) at the bottom.

In a first embodiment of the invention, illustrated in figure 2, a heat exchanger (11) is installed between the feed (6) and the bottom of the VCM column (2). This arrangement leads to a reduction of the energy consumption of the reboiler (10), with a very low impact on the heat duty of the VCM condenser (12).

In a second embodiment of the invention, illustrated in figure 3, a heat exchanger (11) is installed between the feed (6) of the VCM column (2) and the HCl/VCM/EDC mixture coming from the pyrolysis (4) and its downstream quench unit (not shown), right before said mixture enters the second condenser (3'). As can be seen on this figure, not all the mixture coming from the pyrolysis passes through this heat exchanger (11) but instead, some of is by-passed. This arrangement also leads to a reduction of the energy consumption of the reboiler (10).

The embodiments described above have been the object of numerical simulations using ASPEN software, of which you will find the results in tables 1 and 2 below.
Table 1 is the result of a numerical simulation using version V7.2 of the Aspen software and comparing the classical layout (represented in Figure 1) and the layout of Figure 2 using the conditions set forth in said Table 1.
Table 2 is the result of a numerical simulation using version 2004.1 of the Aspen software and comparing the classical layout (represented in Figure 1) and the layout of Figure 3 using the conditions set forth in said Table 2.

As can be seen on these Tables, both the layout of Figure 2 and the one of Figure 3 lead to a substantial reduction of the duty (energy consumption) of the reboiler (10).

**Table 1**

| **FIG 1****.** | **ID** | **4** | **5** | **6** | **7** | **8** | **10** | **12** | | | |
|---|---|---|---|---|---|---|---|---|---|---|---|
| Mass flow rate | kg/s | 27,218 | 6,316 | 20,902 | 10,656 | 10,246 | x | x | | | |
| Volum. flow rate | m3/h | 892,389 | 974,749 | 82,366 | 43,166 | 36,178 | x | x | | | |
| Vapour fraction | kg/kg | 0,19 | 1,00 | 0,00 | 0,00 | 0,00 | x | x | | | |
| Temperature | C | 34,6 | -27,7 | 89,6 | 31,7 | 147,6 | x | x | | | |
| Pressure | bar a | 12,3 | 11,5 | 12,2 | 4,8 | 5,2 | x | x | | | |
| Duty | kW | x | x | x | x | x | 5557,92 | 5821,49 | | | |

| **FIG 2****.** | **ID** | **4** | **5** | **6** | **6'** | **7** | **8** | **8'** | **10** | **11** | **12** |
|---|---|---|---|---|---|---|---|---|---|---|---|
| Mass flow rate | kg/s | 27,22 | 6,32 | 20,90 | 20,90 | 10,66 | 10,25 | 10,25 | x | x | x |
| Volum. flow rate | m3/h | 892,39 | 974,75 | 1307,14 | 1976,58 | 43,17 | 36,18 | 32,44 | x | x | x |
| Vapour fraction | kg/kg | 0,19 | 1,00 | 0,22 | 0,32 | 0,00 | 0,00 | 0,00 | x | x | x |
| Temperature | C | 34,6 | -27,7 | 58,3 | 62,6 | 31,7 | 147,6 | 77,6 | x | x | x |
| Pressure | bar_a | 12,3 | 11,5 | 5,2 | 5,0 | 4,8 | 5,2 | 4,8 | x | x | x |
| Duty | kW | x | x | x | x | x | x | x | 4557,1 | 1012,5 | 5833,3 |

**Table 2**

| **FIG 1****.** | **ID** | **4** | **5** | **6** | **7** | **8** | **10** | **12** | | | | |
|---|---|---|---|---|---|---|---|---|---|---|---|---|
| Mass flow rate | kg/s | 36,57 | 7,85 | 28,98 | 13,23 | 15,75 | x | x | | | | |
| Volum. flow rate | m3/h | 4747,9 | 1212,4 | 112,6 | 53,8 | 55,3 | x | x | | | | |
| Vapour fraction | kg/kg | 1 | 1 | 0 | 0 | 0 | x | x | | | | |
| Temperature | C | 133,3 | -27,7 | 93,5 | 36,7 | 155,6 | x | x | | | | |
| Pressure | bar a | 13,5 | 11,5 | 12,2 | 5,5 | 6,1 | x | x | | | | |
| Duty | kW | x | x | x | x | x | 8079 | 7820 | | | | |

| **FIG 3****.** | **ID** | **4** | **4'** | **4"** | **5** | **6** | **6'** | **7** | **8** | **10** | **11** | **12** |
|---|---|---|---|---|---|---|---|---|---|---|---|---|
| Mass flow rate | kg/s | 36,57 | 8,48 | 8,48 | 7,85 | 28,99 | 28,99 | 13,23 | 15,75 | x | x | x |
| Volum. flow rate | m3/h | 4748,1 | 1101,6 | 791,0 | 1212,4 | 112,7 | 483,2 | 53,8 | 55,3 | x | x | x |
| Vapour fraction | kg/kg | 1,00 | 1,00 | 0,65 | 1,00 | 0,00 | 0,11 | 0,00 | 0,00 | x | x | x |
| Temperature | C | 133,3 | 133,3 | 103,5 | -27,7 | 93,5 | 97,3 | 36,7 | 155,6 | x | x | x |
| Pressure | bar_a | 13,5 | 13,5 | 13,3 | 11,5 | 12,2 | 12,0 | 5,5 | 6,1 | x | x | x |
| Duty | kW | x | x | x | x | x | x | x | x | 6979 | 1100 | 7820 |

## Claims

1. Process for the manufacture of vinyl chloride monomer (VCM), comprising the steps of:
1. subjecting 1,2-dichloroethane (EDC) to pyrolysis in order to generate a gas mixture comprising VCM, HCl and EDC
2. quenching and eventually further cooling and/or condensing said gas mixture to a liquid+gas mixture
3. subjecting said liquid+gas mixture to a first separation step to remove substantially all the HCl there from so as to leave a stream consisting substantially of VCM and EDC
4. subjecting said VCM+EDC stream to a second separation step so as to get a stream of substantially pure VCM and a stream of unconverted EDC,
according to which a heat exchanger is used to heat up the VCM+EDC stream prior to being fed to a distillation column in step 4, said heat exchanger being powered by a stream of hot fluid available in any one of steps 2 to 4 of the process but after the quenching of step 2.

2. Process according to claim 1, wherein the heat exchanger is powered by at least part of the stream of unconverted EDC obtained in step 4.

3. Process according to claim 1, wherein the heat exchanger is powered by a stream of hot mixture comprising VCM, HCl and EDC available in step 2 after the quenching.

4. Process according to any of the preceding claims, wherein in step 2, the gas mixture leaving step 1 is first cooled down in a quench device and thereafter, partially condensed using at least one condenser.

5. Process according to the preceding claim, wherein the quench device uses a liquid quench medium which is a liquid mixture of VCM+HCl+EDC recycled from a downstream condensation step.

6. Process according to claim 4 or 5, wherein at least 2 condensers are used having each an inlet and an outlet stream and wherein the heat exchanger is powered by at least part of the inlet stream of the last condenser.

7. Process according to any of the preceding claims, wherein the first separation step 3 involves a distillation column that separates HCl on top from VCM and EDC at the bottom.

8. Process according to any of the preceding claims, wherein the second separation step 4 involves a distillation column that separates VCM on top while unconverted EDC is purged at the bottom.

9. Process according to any of the preceding claims, wherein the heat exchanger is a multi-tubular heat exchanger, a spiral heat exchanger or a Compabloc® heat exchanger.

10. Process according to claim 9, wherein the heat exchanger is a multi-tubular heat exchanger.

## Patentansprüche

1. Verfahren zur Herstellung von Vinylchloridmonomer (VCM), bei dem man:
1. 1,2-Dichlorethan (EDC) einer Pyrolyse unterwirft, um ein Gasgemisch, das VCM, HCl und EDC umfasst, zu erzeugen,
2. das Gasgemisch abschreckt und schließlich weiter abkühlt und/oder zu einem Flüssigkeit+Gas-Gemisch kondensiert,
3. das Flüssigkeit+Gas-Gemisch einem ersten Trennschritt unterwirft, um weitgehend das gesamte HCl daraus zu entfernen, so dass ein Strom, der weitgehend aus VCM und EDC besteht, zurückbleibt,
4. den VCM+EDC-Strom einem zweiten Trennschritt unterwirft, um einen Strom von weitgehend reinem VCM und einen Strom von nicht umgesetztem EDC zu erhalten,
gemäß dem ein Wärmetauscher zum Erhitzen des VCM+EDC-Stroms vor der Einspeisung in eine Destillationssäule in Schritt 4 verwendet wird, wobei der Wärmetauscher durch einen Strom von heißem Fluid, der in einem der Schritte 2 bis 4 des Verfahrens, aber nach dem Abschrecken von Schritt 2, verfügbar ist, betrieben wird.

2. Verfahren nach Anspruch 1, bei dem der Wärmetauscher durch mindestens einen Teil des Stroms von nicht umgesetztem EDC, der in Schritt 4 erhalten wurde, betrieben wird.

3. Verfahren nach Anspruch 1, bei dem der Wärmetauscher durch einen Strom eines heißen Gemischs, das VCM, HCl und EDC enthält, der in Schritt 2 nach dem Abschrecken verfügbar ist, betrieben wird.

4. Verfahren nach einem der vorhergehenden Ansprüche, bei dem in Schritt 2 das aus Schritt 1 austretende Gasgemisch zunächst in einer Abschreckungsvorrichtung abgekühlt und danach unter Verwendung mindestens eines Kondensators teilweise kondensiert wird.

5. Verfahren nach dem vorhergehenden Anspruch, bei dem in der Abschreckungsvorrichtung ein flüssiges Abschreckungsmedium verwendet wird, bei dem es sich um ein aus einem nachgeschalteten Kondensationsschritt rezykliertes flüssiges Gemisch von VCM+HCl+EDC handelt.

6. Verfahren nach Anspruch 4 oder 5, bei dem mindestens 2 Kondensatoren verwendet werden, die jeweils einen Einlass- und einen Auslassstrom aufweisen, und wobei der Wärmetauscher durch mindestens einen Teil des Einlassstroms des letzten Kondensators betrieben wird.

7. Verfahren nach einem der vorhergehenden Ansprüche, bei dem im ersten Trennschritt 3 eine Destillationssäule verwendet wird, die HCl am Kopf von VCM und EDC am Sumpf trennt.

8. Verfahren nach einem der vorhergehenden Ansprüche, bei dem im zweiten Trennschritt 4 eine Destillationssäule verwendet wird, die VCM am Kopf abtrennt, während nicht umgesetztes EDC am Sumpf abgelassen wird.

9. Verfahren nach einem der vorhergehenden Ansprüche, bei dem es sich bei dem Wärmetauscher um einen Multirohrwärmetauscher, einen Spiralwärmetauscher oder einen Compabloc®-Wärmetauscher handelt.

10. Verfahren nach Anspruch 9, bei dem es sich bei dem Wärmetauscher um einen Multirohrwärmetauscher handelt.

## Revendications

1. Procédé de fabrication de monomère de chlorure de vinyle (VCM), comprenant les étapes consistant à :
1. soumettre du 1,2-dichloroéthane (EDC) à une pyrolyse afin de générer un mélange gazeux comprenant du VCM, du HCl et de l'EDC
2. tremper et optionnellement refroidir encore et/ou condenser ledit mélange gazeux en un mélange liquide+gaz
3. soumettre ledit mélange liquide+gaz à une première étape de séparation pour en retirer pratiquement tout le HCl de manière à laisser un courant essentiellement composé de VCM et d'EDC
4. soumettre ledit courant de VCM+EDC à une deuxième étape de séparation de manière à obtenir un courant de VCM sensiblement pur et un courant d'EDC non transformé,
selon lequel un échangeur de chaleur est utilisé pour chauffer le courant de VCM+EDC avant qu'il soit introduit dans une colonne de distillation à l'étape 4, ledit échangeur de chaleur étant alimenté par un courant de fluide chaud disponible dans l'une quelconque des étapes 2 à 4 du procédé, mais après la trempe de l'étape 2.

2. Procédé selon la revendication 1, dans lequel l'échangeur de chaleur est alimenté par au moins une partie du courant d'EDC non transformé obtenu à l'étape 4.

3. Procédé selon la revendication 1, dans lequel l'échangeur de chaleur est alimenté par un courant de mélange chaud comprenant du VCM, du HCl et de l'EDC disponible à l'étape 2 après la trempe.

4. Procédé selon l'une quelconque des revendications précédentes, dans lequel à l'étape 2, le mélange gazeux quittant l'étape 1 est d'abord refroidi dans un dispositif de trempe et ensuite partiellement condensé en utilisant au moins un condenseur.

5. Procédé selon la revendication précédente, dans lequel le dispositif de trempe utilise un milieu de trempe liquide qui est un mélange liquide de VCM+HCl+EDC recyclé à partir d'une étape de condensation aval.

6. Procédé selon la revendication 4 ou 5, dans lequel au moins 2 condenseurs sont utilisés, chacun ayant un courant d'entrée et de sortie, et dans lequel l'échangeur de chaleur est alimenté par au moins une partie du courant d'entrée du dernier condenseur.

7. Procédé selon l'une quelconque des revendications précédentes, dans lequel la première étape de séparation 3 implique une colonne de distillation qui sépare le HCl au sommet du VCM et de l'EDC au fond.

8. Procédé selon l'une quelconque des revendications précédentes, dans lequel la deuxième étape de séparation 4 implique une colonne de distillation qui sépare le VCM au sommet tandis que l'EDC non transformé est purgé au fond.

9. Procédé selon l'une quelconque des revendications précédentes, dans lequel l'échangeur de chaleur est un échangeur de chaleur multitubulaire, un échangeur de chaleur à serpentin ou un échangeur de chaleur Compabloc®.

10. Procédé selon la revendication 9, dans lequel l'échangeur de chaleur est un échangeur de chaleur multitubulaire.
